# EUROPEAN PATENT APPLICATION

(11) **EP 1 284 421 A1**
(43) Date of publication of application: **19.02.2003**
(21) Application number: 01930028.4
(22) Date of filing: 10.05.2001
(51) Int. Cl.: G01N 33/53, G01N 33/566

(54) **METHOD OF CONSTRUCTING POLYNUCLEOTIDE MICROARRAY, APPARATUS FOR THE CONSTRUCTION AND POLYNUCLEOTIDE MICROARRAY**

(30) Priority: 12.05.2000 JP 2000139926
(71) Applicant: KYOWA HAKKO KOGYO CO., LTD., Chiyoda-ku, Tokyo 100-8185 (JP); Nippon Laser & Electronics Lab., Nagoya-shi, Aichi 456-0032 (JP); Watanabe, Shinya, Tokyo 108-0071 (JP)
(72) Inventor: WATANABE, Shinya, Minato-ku, Tokyo 108-0071 (JP)
(74) Representative: Sternagel, Fleischer, Godemeyer & Partner Patentanwälte
(86) International application number: JP0103899
(87) International publication number: WO01086294

(57) **Abstract**

An object of the present invention is to provide a method for preparing a polynucleotide microarray which enables fixation of a sufficient amount of polynucleotides onto the support and can provide signals having uniform and sufficient intensity within a spot area, even when the polynucleotides are synthetic oligo polynucleotides which have a short chain length and are known to be difficult to be fixed by electrostatic binding. The present invention provides a method for preparing a polynucleotide microarray, wherein a solution for dissolving polynucleotides which is used to fix the polynucleotides onto a support contains substantially no anionic electrolyte, and is a solution of water or mixture of water and an organic solvent; and when the solution is a mixture of water and an organic solvent, the content of the organic solvent in the solution is less than 50 volume %.

## Description

### TECHNICAL FIELD

The present invention relates to a method for preparing a polynucleotide microarray, a device for preparing a polynucleotide microarray which is used in the performance of the method, and the polynucleotide microarray prepared by the method.

### BACKGROUND ART

Devices for preparing polynucleotide microarrays are marketed by many companies (GeneTip Microarray Stamping System manufactured by Nippon Laser & Electronics Lab., PixSys 5200/5500 manufactured by Cartesian Technologies, Q-array manufactured by Genetix, Omni Grid manufactured by GeneMachines, 417 Arrayer manufactured by Affymetrix, MicroGrid manufactured by BioRobotics, SPBIO2000 manufactured by Hitachi Software Engineering and the like). These devices are used for analyzing the expression profiles and mutations of many numbers of genes at the same time.

A method which is widely and generally used for preparing a polynucleotide microarray is a method of fixing polynucleotide molecules onto a positively-charged support such as a slide glass whose surface has been treated with poly-L-lysine or the like.

In the conventional method for fixing polynucleotides, a polynucleotide samples dissolved in a solution containing appropriate salts such as 3 x SSC (0.45 mol/L sodium chloride, 0.045 mol/L sodium citrate, pH 7.0) are spotted under room temperature condition onto a positively-charged glass plate whose surface has been treated with poly-L-lysine or the like, thereby fixing polynucleotide molecules onto the glass surface [Science, 20, 467 (1995); and Proc. Natl. Acad. Sci. USA., 1, 10614 (1996)].

The conventional method for fixing polynucleotides as mentioned above has problems such that when the evaporation rate of a solution containing polynucleotides dissolved therein is too high, the solvent gradually evaporates during the spotting of the polynucleotide samples and the concentration of the polynucleotide solution changes, and the samples become dried and solidified on the pin or pen that is used for spotting the polynucleotide samples, or the concentration of the polynucleotide sample within a spot area after spotting becomes uneven. Therefore, it has been difficult to prepare a polynucleotide microarray which is fully satisfactory for the subsequent analysis.

To solve the above-mentioned problems, it is considered to be important to control the evaporation rate of a solution for dissolving polynucleotides. However, this cannot completely solve the problem that the concentration of polynucleotide sample within a spot area becomes uneven.

Further, in the conventional method, the amount of polynucleotide molecules to be fixed onto a glass surface is small, and sufficient signal strength cannot be obtained. Therefore, the conventional method is not appropriate for preparing a polynucleotide microarray onto which oligonucleotide molecules having a relatively short chain length of 70 or less nucleotides are fixed.

As compared with a case in which PCR amplified products are fixed, it is difficult in the conventional method to firmly fix a sufficient amount of oligo DNAs. Therefore, it has been attempted to increase the amount of polynucleotides to be fixed onto a glass surface by, for example, increasing the concentration of a polynucleotide sample to be spotted. Since the concentration of a polynucleotide solution which can be prepared is limited, it has been difficult even with such an attempt to prepare a polynucleotide microarray onto which polynucleotides of short chain length were spotted and which can provide sufficient signal strength.

### DISCLOSURE OF THE INVENTION

An object to be attained by the present invention is to provide a method for preparing a polynucleotide microarray which enables fixation of a sufficient amount of polynucleotides onto the support and can provide signals having uniform and sufficient intensity within a spot area, even when the polynucleotides are synthetic oligo polynucleotides which have a short chain length and are known to be difficult to be fixed by electrostatic binding.

Another object to be attained by the present invention is to provide a device for preparing a polynucleotide microarray to be used in the above-mentioned method of the present invention, and a polynucleotide microarray prepared by the above-mentioned method of the present invention.

The present inventors made an intensive investigation into the method for preparing a polynucleotide microarray in order to attain the above objects. As a result, the present inventors have found that a polynucleotide microarray which can provide uniform and sufficiently strong signals within a spot area can be prepared even in the case of synthetic oligo polynucleotides of short chain length, by fixing polynucleotides onto a support by using a solution which contains substantially no anionic electrolyte and is a solution of water or mixture of water and an organic solvent as a solution for dissolving the polynucleotides, thereby completing the present invention.

Thus, the present invention provides the following (1) to (12):
(1) A method for preparing a polynucleotide microarray, wherein a solution for dissolving polynucleotides which is used to fix the polynucleotides onto a support contains substantially no anionic electrolyte, and is a solution of water or mixture of water and an organic solvent; and when the solution is a mixture of water and an organic solvent, the content of the organic solvent in the solution is less than 50 volume %.
(2) The method according to (1), wherein the solution of water or mixture of water and an organic solvent contains 0 to 0.1 mol/L anionic electrolytes.
(3) The method according to (1) or (2), wherein polynucleotides are dissolved in a solution of mixture of water and an organic solvent, and the content of the organic solvent in the solution is less than 50 volume %.
(4) The method according to (1) or (2), wherein the polynucleotides are dissolved in an aqueous solution.
(5) The method according to any of (1) to (3), wherein the polynucleotides are dissolved in a solution containing dimethylsulfoxide of less than 20 volume %.
(6) The method according to any of (1) to (5), wherein the solution containing substantially no anionic electrolyte has a vapor pressure of 12 to 100 hPa at 20°C.
(7) The method according to any of (1) to (6), wherein the concentration of polynucleotides in a polynucleotide solution which is obtained by dissolving polynucleotides in a solution containing substantially no anionic electrolyte is 0.01 to 2.0 µg/µl.
(8) The method according to any of (1) to (7), wherein the polynucleotide which is fixed onto a support is a polynucleotide of 12 to 120 nucleotides in length.
(9) The method according to any of (1) to (8), wherein the support has a positive charge.
(10) The method according to any of (1) to (9), which comprises spotting a solution in which polynucleotides to be fixed onto support are dissolved, onto the support under a temperature condition of 0 to 10°C.
(11) A device for preparing a polynucleotide microarray, which is used for conducting the method according to any of (1) to (10) and has a temperature controlling function.
(12) A polynucleotide microarray, which is prepared by the method of any of (1) to (10).

### BRIEF DESCRIPTION OF DRAWINGS

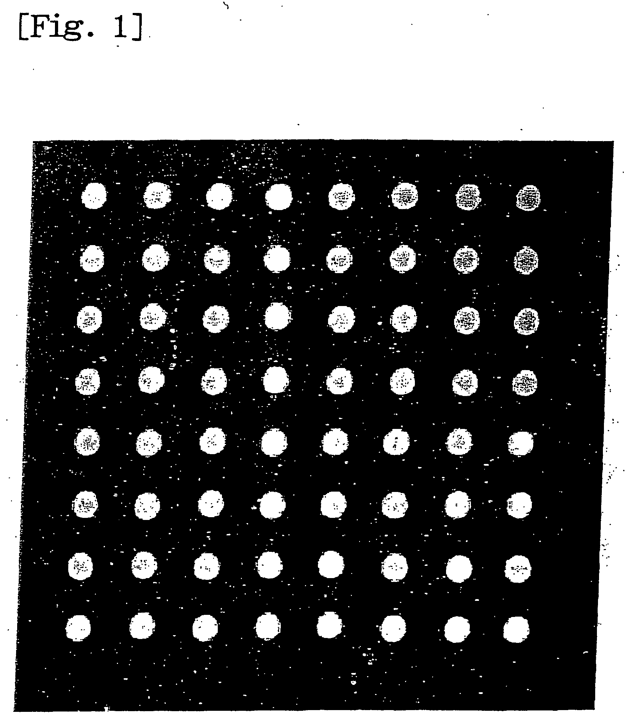

Figure. 1 shows a hybridization image in DNA microarray prepared by the method of the present invention. DNA microarray prepared by the method described in Example 1 is used, hybridization and washing were performed according to the methods described in Examples 2 to 5, and imaging was performed using a scanner according to the method described in Example 6.

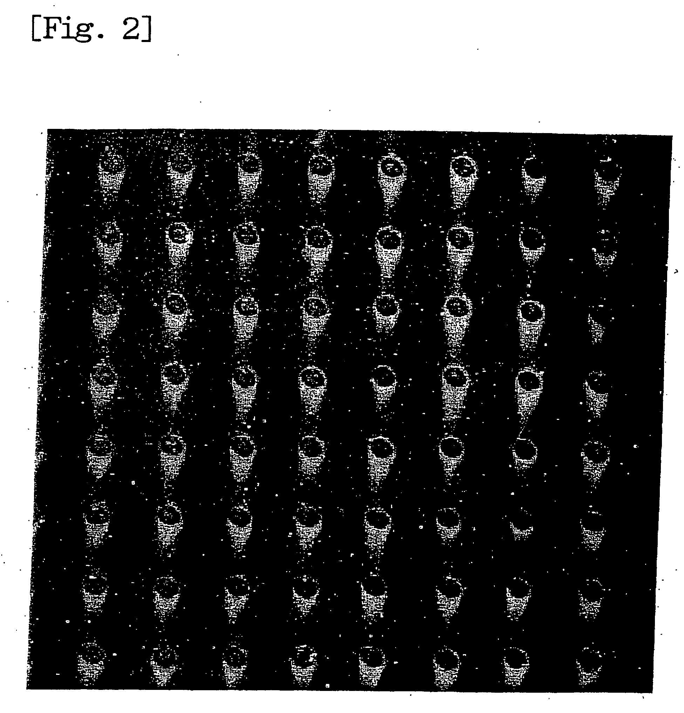

Figure 2 shows a hybridization image in DNA microarray prepared by spotting DNA samples dissolved in 3 x SSC solution under a temperature condition of 22°C. DNA microarray prepared by the method described in Comparative Example 1 is used, hybridization and washing were performed according to the methods described in Examples 2 to 5, and imaging was performed using a scanner according to the method described in Example 6.

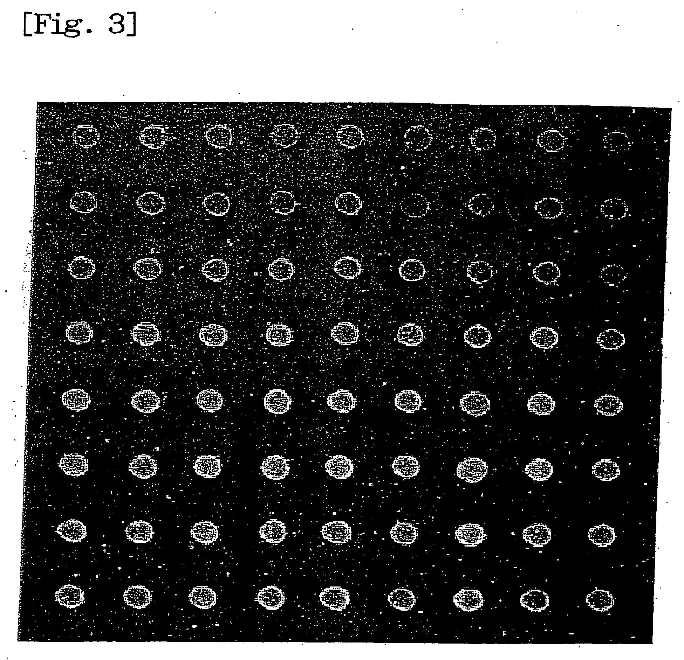

Figure 3 shows a hybridization image in DNA microarray prepared by spotting DNA samples dissolved in 50% DMSO solution under a temperature condition of 22°C. DNA microarray prepared by the method described in Comparative Example 2 is used, hybridization and washing were performed according to the methods described in Examples 2 to 5, and imaging was performed using a scanner according to the method described in Example 6.

### BEST MODE FOR CARRYING OUT THE INVENTION

Hereinafter, methods for carrying out the present invention and embodiments of the present invention will be described in detail.

The method for preparing a polynucleotide microarray according to the present invention is a method which comprises a step of fixing polynucleotides dissolved in a solution of water or mixture of water and an organic solvent onto a support, wherein the solution contains substantially no anionic electrolyte, and when the solution is a mixture of water and an organic solvent, the content of the organic solvent in the solution is less than 50 volume %.

Hereinafter, (A) preparation of a polynucleotide solution for spotting and (B) spotting of polynucleotides onto a support are described.

### (A) Preparation of polynucleotide solution for fixation

The types of polynucleotides (also referred to as nucleic acids) used in the present invention are not particularly limited, and examples thereof include deoxyribonucleic acid (DNA) and ribonucleic acid (RNA). The types of DNA and RNA are also not particularly limited. For example, any of DNA fragment derived from chromosomal DNA, mRNA, cDNA, chemically synthesized DNA and chemically synthesized RNA may be used. More specifically, any of PCR amplified product, DNA cleaved with restriction enzyme, chemically synthesized DNA, chemically synthesized RNA and the like may be used. In the method of the present invention, synthetic oligo DNA having a length of 12 to 120 nucleotides can be efficiently used as a chemically synthesized DNA.

As a polynucleotide used in the present invention, those derived from any organism can be used. Examples of the polynucleotide used in the present invention include a DNA fragment, a cDNA fragment and chemically synthesized DNA, which are derived from chromosomal locus known to associate with diseases. Further, the polynucleotide may be, for example, a DNA fragment containing a part of a certain gene, or mRNA showing abnormal transcription level, or cDNA derived from such mRNA.

The solution used for dissolving polynucleotides in the present invention is characterized in that a solution contains substantially no anionic electrolyte, and when the solution is a mixture of water and an organic solvent, the content of the organic solvent in the solution is less than 50 volume %.

The phrase "a solution contains substantially no anionic electrolyte" refers not only to a solution containing no anionic electrolyte, but also to a solution containing anionic electrolytes in such an amount that, when polynucleotides are dissolved in this solution and are spotted onto a support such as a glass plate, the electrostatic binding of the polynucleotides to the surface of the support is not prevented and good spot images can be obtained.

The concentration of total anionic electrolytes is preferably 0 to 0.1 mol/L, and more preferably 0 to 0.01 mol/L.

The above-mentioned solution which substantially contains no anionic electrolyte can be prepared by adjusting the concentration of salts to be dissolved in a solvent.

As a solvent for the solution which substantially contains no anionic electrolyte, any solvent can be used, so long as polynucleotides can be dissolved therein. Preferred examples of the solvent include water, and a mixed solvent of water and an organic solvent. Examples of the organic solvent include an organic solvent which can be mixed with water at any ratio, such as ethanol, methanol or dimethylsulfoxide (DMSO). The content of the organic solvent in a mixed solvent of water and an organic solvent is less than 50 volume %, preferably 30 or less volume %, and more preferably 20 or less volume %.

Further, the above-mentioned solution for dissolving polynucleotides is preferably a solution having a vapor pressure of 12 to 100 hPa at 20 °C. For an aqueous solvent, the vapor pressure of the solution can be adjusted by adding an organic solvent such as methanol, ethanol or DMSO.

The concentration of polynucleotide in a polynucleotide solution is preferably 0.01 to 2.0 µg/µl, and more preferably 0.15 to 0.30 µg/µl as a final concentration.

### (B) Spotting of polynucleotide

In the method of the present invention, the polynucleotide solution prepared in (A) above is spotted onto a support (preferably, a positively-charged support).

Examples of a support include glass, membrane and plastic. A variety of natural or synthetic organic polymers and inorganic polymers can be used as materials for the solid surface of the support. Examples of solid surfaces of the support include nitro cellulose, nylon, glass, diazotized membrane (paper or nylon), silicone, polyformaldehyde, cellulose or cellulose acetate. Alternatively, plastic (for example, polyethylene, polypropylene, polystyrene and the like) can be used. When the solid surface of the support is porous, a support having various pore sizes can be used appropriately.

As the positively-charged support as mentioned above, a commercially available support can be used. For example, a slide glass manufactured by MATSUNAMI GLASS IND.(model S9115) can be used.

When the solid surface of a support is prepared, several different materials can be used, for example, as laminate. For example, signal detection can be increased using a mixture of protein (for example, bovine serum albumin) and macromolecules (for example, Denhardt's solution) as laminate.

The temperature environment under which a polynucleotide solution is spotted onto a support is important. When a polynucleotide solution having a low vapor pressure at 20 °C is used, spotting onto a support must be performed at a low temperature. For example, when a polynucleotide solution prepared using water is used, spotting is performed under a temperature environment of preferably 0°C to 10 °C, and more preferably 2°C to 6°C. It is undesirable to perform spotting at a high temperature such as room temperature, because the evaporation rate of water becomes too high and uneven spotting occurs.

Spotting of polynucleotides onto a support is performed by attaching a polynucleotide solution to a pin or a pen for preparing a DNA microarray, and allowing the pin or the pen to be in contact with the surface of the support preferably having a positive charge.

The present inventors have found for the first time that, in a method wherein the oligonucleotide sample obtained by dissolving oligonucleotides in a solvent containing salts such as 3 x SSC solution is spotted as in the conventional method, anionic electrolytes other than oligonucleotides which are contained in the solvent for dissolving the oligonucleotides inhibit fixation of the oligonucleotides onto the support. In addition, it is also the fact which has been found for the first time in the present invention that the evaporation rate of an oligonucleotide solution can be controlled by temperature.

The spot size in an oligonucleotide microarray prepared by the method of the present invention is not particularly limited. Generally, the size is less than 1cm in diameter, and for example, ranges from 1 µm to 3 mm, and more preferably 5 µm to 1 mm.

Further, the spot density in an oligonucleotide microarray prepared by the method of the present invention is not particularly limited. Generally, the density ranges from 5 to 20,000 spots, preferably 10 to 10,000 spots, and more preferably 100 to 8,000 spots per 1cm².

There may be a case wherein highly dense spotting of oligonucleotides onto a support is preferred. For example, when the nucleotide sequence of nucleic acid is determined using an oligonucleotide microarray, it is preferred to arrange nucleic acid probes of as many types as possible on a support. Further, in the case of detecting gene mutations efficiently, it is preferred to arrange oligonucleotide probes having sequences corresponding to each mutation on a solid phase. Furthermore, in the cases of detecting a target oligonucleotide in a sample, or detecting gene mutation or deletion, it is preferred to minimize the amount of samples from a subject, specifically blood or the like. Therefore, it is preferred to obtain the maximum information on the nucleotide sequences using the minimum amount of analytes. From this view point, it is preferred to spot oligonucleotides onto a microarray at the density which is as high as possible.

Even in the case of an oligonucleotide having a chain length of 20 nucleotides or less which has been thought to be difficult to be fixed by electrostatic binding because of its short chain length, spotting by the above-mentioned method of the present invention allows the preparation of an oligonucleotide microarray wherein a uniform and sufficient strength signal can be obtained within a spot area.

Fixation of polynucleotides onto a support can be carried out by a conventional method, for example, a method based on the following procedure.

The support to which polynucleotides are spotted is put in a gas phase incubator, and is incubated at 80°C for 1 hour. After incubation, ultraviolet rays are irradiated using a UV crosslinker. Polynucleotide molecules can be fixed onto the surface of the support by this procedure.

Using the thus obtained polynucleotide-fixed support, hybridization with fluorescence-labeled oligonucleotide probes is performed according to a conventional method, such as that reported by M. Schena et al, [Science, 20, 467 (1995)]. Then, the support is washed, and measurement is carried out using a fluorescent scanner, so that hybridization between fixed polynucleotides and fluorescence-labeled oligonucleotides can be detected.

As a label for an oligonucleotide, an enzyme label can also be used instead of a fluorescence label. Examples of an enzyme using an enzyme label include enzymes which are normally used for ELISA. Specific examples of an enzyme using an enzyme label include horseradish peroxidase (HRP) and alkaline phosphatase (ALP). When an enzyme label is used, hybridization can be detected by adding an enzyme substrate which gives a detectable signal after hybridization.

Generally, it is preferred that the label is detectable even when the copy number of the target oligonucleotide is small, thereby can improve the sensitivity of assay, and is detectable regardless of the presence of background signals.

An example of a device for preparing the polynucleotide microarray of the present invention is one having a temperature controlling function which enables spotting of polynucleotides at any temperature such as a temperature condition of preferably 0°C to 10°C, and more preferably 2°C to 6°C. A specific example of such a device is a device for preparing a polynucleotide microarray which is so designed that at least an area including a pin for spotting polynucleotides which is provided with the device and a support onto which polynucleotides are spotted is controlled at the above-mentioned temperature. A more specific example is a device wherein the above-mentioned area is partitioned off with fresh air, and a temperature sensor such as a thermostat is provided in the partitioned inside area, and which has a function of heating and cooling the area to allow spotting to be performed at a set temperature.

The polynucleotide microarray of the present invention can be prepared using the above-mentioned device for preparing the polynucleotide microarray of the present invention. Moreover, the polynucleotide microarray of the present invention can also be prepared by applying the above-mentioned method of the present invention using a general commercially available device for preparing a DNA microarray, such as GeneTip Microarray Stamping System manufactured by Nippon Laser Electronics Lab., PixSys 5200/5500 manufactured by Cartesian Technologies, Q-array manufactured by Genetix, Omni Grid manufactured by GeneMachines, 417 Arrayer manufactured by Affymetrix, MicroGrid manufactured by BioRobotics, SPBIO2000 manufactured by Hitachi Software Engineering or the like, in accordance with a conventional method such as methods described in manuals attached to these devices.

The above-mentioned commercially available devices do not have a temperature controlling function. Thus, if necessary, spotting is performed after putting the device in a temperature-controllable low temperature room or refrigerator, so that the polynucleotide microarray of the present invention can be prepared.

The present invention is explained by the following examples more specifically, but the present invention is not limited by these examples.

### Example

### (Example 1) Preparation of DNA microarray by the method of the present invention

DNA microarray was prepared by the following methods. Synthetic oligo DNA having a nucleotide sequence of SEQ ID NO: 1 was dissolved in water at 0.2 µg/µL (vapor pressure at 20°C is 23.379 hPa). DNA sample was spotted onto a slide glass manufactured by MATSUNAMI GLASS IND. (model S9115) under conditions which allow the DNA sample to be in contact with the glass surface at 4°C for 0.5 seconds using an arrayer manufactured by Nippon Laser & Electronics Lab. (GTMASS stamping) and a pin manufactured by Nippon Laser & Electronics Lab (the type with 150 µm in diameter). The thus obtained slide glass onto which the DNA sample was spotted was left and incubated in a gas phase incubator (manufactured by Stovall Life Science, Hybridization Oven) at 80°C for 1 hour. Then, 120mJ of ultra-violet rays were irradiated at the slide glass using a UV cross linker (manufactured by Hoefer, UVC 500), thereby fixing the spotted synthetic oligo DNA onto the surface of the slide glass.

### (Comparative Example 1) Preparation of DNA microarray by a method of dissolving DNA sample in 3 x SSC

DNA microarray was prepared by the following methods. Synthetic oligo DNA having a nucleotide sequence of SEQ ID NO: 1 was dissolved in 3 x SSC solution (0.45M sodium chloride, 0.045M sodium citrate, pH 7.0) at 0.2 µg/µL. DNA sample was spotted onto a slide glass manufactured by MATSUNAMI GLASS IND. (model S9115) under conditions which allow the DNA sample to be in contact with the glass surface at 22°C for 0.5 seconds using an arrayer manufactured by Nippon Laser & Electronics Lab. (GTMASS stamping) and a pin manufactured by Nippon Laser & Electronics Lab (the type with 150 µ m in diameter). The thus obtained slide glass onto which the DNA sample was spotted was left and incubated in a gas phase incubator (manufactured by Stovall Life Science, Hybridization Oven) at 80°C for 1 hour. Then, 120mJ of ultra-violet rays were irradiated at the slide glass using a UV cross linker (manufactured by Hoefer, UVC 500), thereby fixing the spotted synthetic oligo DNA onto the surface of the slide glass.

### (Comparative Example 2) Preparation of DNA microarray by a method of dissolving DNA sample in DMSO solution of 50 volume %

DNA microarray was prepared by the following methods. Synthetic oligo DNA having a nucleotide sequence of SEQ ID NO: 1 was dissolved in DMSO solution of 50 volume % (dimethyl sulfoxide, manufactured by Wako Pure Chemicals Industries, Ltd., #043-07216) (vapor pressure at 20°C: 11.98hPa) at 0.2 µg/µL. DNA sample was spotted onto a slide glass manufactured by MATSUNAMI GLASS IND. (model S9115) under conditions which allow the DNA sample to be in contact with the glass surface at 22°C for 0.5 seconds using an arrayer manufactured by Nippon Laser & Electronics Lab. (GTMASS stamping) and a pin manufactured by Nippon Laser & Electronics Lab (the type with 150 µ m in diameter). The thus obtained slide glass onto which the DNA sample was spotted was left and incubated in a gas phase incubator (manufactured by Stovall Life Science, Hybridization Oven) at 80°C for 1 hour. Then, 120mJ of ultra-violet rays were irradiated at the slide glass using a UV cross linker (manufactured by Hoefer, UVC 500), thereby fixing the spotted synthetic oligo DNA onto the surface of the slide glass.

### (Example 2) Blocking of a slide glass surface

The slide glasses to which DNA sample was spotted as described in Example 1 and Comparative Examples 1 and 2, were immersed in a blocking solution [the solution consists of 5g of succinic anhydride (manufactured by Aldrich, 23969-0), 315ml of N-methyl-2-pyrrolidinone (manufactured by Aldrich, 32863-4), and 35ml of 0.2M sodium borate (pH 8.0)], shaken up and down intensely several dozen times, and then incubated at room temperature for 1 hour with slow shaking. The slide glasses which were taken out from the solution were soaked into hot water at 95 °C to 99°C, shaken up and down intensely for 1 minute, and then soaked in hot water as it was. Then, the whole container including the slide glass was put into a box made of styrene-foam, and allowed to stand for 1 hour with keeping the temperature. After putting the slide glasses in and out of 99% ethanol three times, the slide glasses were taken out, and air-dried at room temperature.

### (Example 3) Preparation of fluorescent labeling probe

Synthetic oligo DNA of a nucleotide sequence of SEQ ID NO: 2 labeled with fluorescent dye Cy3 or Cy5 (manufactured by Amersham Pharmacia Biotech, #PA23000, #PA25000) of its 5' terminus was custom synthesized by and purchased from Greiner Japan. These labeled oligo DNAs were adjusted to have a concentration of 0.1 µg/µL. 0.5 µL of each labeled oligo DNA was mixed with 14 µL of a hybridization buffer (manufactured by Ambion, # 8670), and then left and incubated at 68°C for 15 minutes.

### (Example 4) Hybridization

The probe solution prepared in Example 3 was dropped onto regions on the slide glasses prepared in Example 2 where the DNA sample was spotted. Then, the slide glasses were covered with cover glasses of 24x24mm (manufactured by MATSUNAMI GLASS IND., Microcoverglass No.1). The slide glasses were put into a hybridization cassette (manufactured by Telechem, THC-1), and left and incubated at 68°C in a gas phase incubator (manufactured by Stovall Life Science, Hybridization Oven) for 15 hours.

### (Example 5) Washing

The slide glasses were taken out from the gas phase incubator, and then immersed gently in 200ml of 2 x SSC solution (0.3M sodium chloride, 0.03M sodium citrate, pH 7.0) containing 0.1% SDS (sodium dodecyl sulfate), and then the cover glasses were peeled off. The slide glasses from which the cover glasses had been removed were immersed in 200ml of 1 x SSC solution at room temperature for 3 minutes, and then immersed in 0.2xSSC solution at room temperature for 3 minutes for washing. The slide glasses were then put in a slide glass holder, and centrifuged at 500 rpm for 10 seconds using a low speed centrifugal machine (manufactured by Sakuma Seisakusho, LTD., M200-IVD) to remove moisture.

### (Example 6) Detection and imaging of hybridization

The fluorescence signals at wavelengths of 635nm and 532nm of the thus obtained slide glasses after hybridization and washing were simultaneously measured using a scanner (manufactured by Axon Instruments, GenePix4000), and each spot was imaged on the same screen. The results are shown in Fig. 1 (DNA microarray prepared in Example 1 was used), Fig. 2 (DNA microarray prepared in Comparative Example 1 was used) and Fig. 3 (DNA microarray prepared in Comparative Example 2 was used). As is understood from the results of Figs.1 to 3, the signal strength is significantly higher in the case of using the method of the present invention than in the case of the Comparative Examples.

### Industrial Applicability

According to the present invention, significantly enhanced signal intensity is detected when oligo DNA having a short chain length of 20 nucleotides is spotted on a glass plate, and uneven signal intensity within a measurement area, which is caused by the loss of a part of the spotted DNA sample during processes subsequent to spotting, was reduced. As a result, fluctuation of measurement in signal detection after hybridization can also be reduced.

## Claims

1. A method for preparing a polynucleotide microarray, wherein a solution for dissolving polynucleotides which is used to fix the polynucleotides onto a support contains substantially no anionic electrolyte, and is a solution of water or mixture of water and an organic solvent; and when the solution is a mixture of water and an organic solvent, the content of the organic solvent in the solution is less than 50 volume %.

2. The method according to claim 1, wherein the solution of water or mixture of water and an organic solvent contains 0 to 0.1 mol/L anionic electrolytes.

3. The method according to claim 1 or 2, wherein polynucleotides are dissolved in a solution of mixture of water and an organic solvent, and the content of the organic solvent in the solution is less than 50 volume %.

4. The method according to claim 1 or 2, wherein the polynucleotides are dissolved in an aqueous solution.

5. The method according to any one of claims 1 to 3, wherein the polynucleotides are dissolved in a solution containing dimethylsulfoxide of less than 20 volume %.

6. The method according to any one of claims 1 to 5, wherein the solution containing substantially no anionic electrolyte has a vapor pressure of 12 to 100 hPa at 20°C.

7. The method according to any one of claims 1 to 6, wherein the concentration of polynucleotides in a polynucleotide solution which is obtained by dissolving polynucleotides in a solution containing substantially no anionic electrolyte is 0.01 to 2.0 µg/ µl.

8. The method according to any one of claims 1 to 7, wherein the polynucleotide which is fixed onto a support is a polynucleotide of 12 to 120 nucleotides in length.

9. The method according to any one of claims 1 to 8, wherein the support has a positive charge.

10. The method according to any one of claims 1 to 9, which comprises spotting a solution in which polynucleotides to be fixed onto support are dissolved, onto the support under a temperature condition of 0 to 10°C.

11. A device for preparing a polynucleotide microarray, which is used for conducting the method according to any one of claims 1 to 10 and has a temperature controlling function.

12. A polynucleotide microarray, which is prepared by the method of any one of claims 1 to 10.
